# EUROPEAN PATENT APPLICATION

(11) **EP 2 215 998 A1**
(43) Date of publication of application: **11.08.2010**
(21) Application number: 08849827.4
(22) Date of filing: 16.09.2008
(51) Int. Cl.: A61F 13/15, A61F 13/49, A61F 13/58

(54) **DISPOSABLE DIAPER**

(30) Priority: 15.11.2007 JP 2007297301; 15.11.2007 JP 2007297302
(71) Applicant: Unicharm Corporation, Ehime 799-0111 (JP)
(72) Inventor: SHIMADA, Takaaki, Kanonji-shi Kagawa 769-1602 (JP); YAGI, Akiko, Kanonji-shi Kagawa 769-1602 (JP); MAKI, Hideaki, Kanonji-shi Kagawa 769-1602 (JP)
(74) Representative: Peter, Julian
(86) International application number: PCT/JP2008/066676
(87) International publication number: WO 2009/063687

(57) **Abstract**

The present invention on its first aspect provides a disposable diaper improved so as to prevent body waste from leaking sideways without sacrificing a bodily fluid absorbing capacity of a liquid-absorbent structure and to assure appropriate fitness to the wearer's body.

In each of front and rear waist regions 13, 14, a disposable diaper 10 comprises an upper elasticized region 18 extending in a vicinity of a waist-opening periphery 16a, lower elasticized regions 20A, 20B, intermediate elasticized region 19 extending between the upper elasticized region 18 and the lower elasticized regions 20A, 20B and outside longitudinally opposite ends 12a, 12b of a liquid-absorbent structure 12, and, at least in the front waist region 13 of the front and rear waist regions 13, 14, a non-elasticized region 30 opposed to a central zone of a liquid-absorbent core wherein a tensile stress of the intermediate elasticized region 19 is lower than a tensile stress of the upper elasticized region 18.

## Description

### TECHNICAL FIELD

The present invention comprises a first aspect and a second aspect. The first aspect of the present invention relates to a disposable diaper and particularly to a disposable diaper having a good fit to the wearer's body. The second aspect of the present invention relates to a disposable diaper and particularly to a disposable diaper adapted to prevent bodily fluids from leaking sideways and to protect the wearer's body from soiling due to defecation without sacrificing a desired absorption capacity.

### RELATED ART

There have already been proposed disposable diapers provided in a front waist region as well as in a rear waist region with a plurality of elastic members circumferentially extending therein so that these elastic members may serve to improve a fit of the diaper to the wearer' s body. For example, JP 2002-248127A cited herein discloses a disposable diaper comprising an absorbent structure extending across a crotch region and further extending into front and rear waist regions and a plurality of elastic members extending from transversely opposite side edges of the respective waist regions to at least outer side edges of the absorbent structure in a transverse direction.
PATENT DOCUMENT 1: JP 2002-248127A

### DISCLOSURE OF THE INVENTION

### PROBLEM TO BE SOLVED BY THE INVENTION

### <First Aspect of the Invention>

The elastic members or the waist regions in the diaper disclosed by JP 2002-248127A comprise a plurality of elastic members circumferentially extending completely along respective waist-opening peripheries of the front and rear waist regions and a plurality of elastic members extending from the side edges of the respective waist regions to the side edges of the absorbent structure. In this way, a tensile stress of the waist elastic members is exerted on the side edges of the absorbent structure so as to improve a fit of the absorbent structure to the wearer's body.

According to the disclosure of JP 2002-248127A, the elastic members associated with the waist regions starting from the side edges of the respective waist regions do not extend across a central zone of the absorbent structure but terminate on lateral regions of the absorbent structure. Consequently, the tensile stress of these elastic members should not be directly exerted on the central zone of the absorbent structure and significantly affect the absorption capacity of the absorbent structure.

However, some of these elastic members associated with the waist regions circumferentially extend just above a pair of longitudinally opposite ends of the absorbent structure and the central zone of the absorbent structure should get wrinkled under contraction of these elastic members and the absorption capacity of the absorbent structure should be deteriorated. Obviously it may be contemplated to partially cut the elastic members extending above these ends of the absorbent structure or to partially remove these elastic members. However, according to such a countermeasure the regions in which none of the elastic members is present should drop down between the absorbent structure and the regions in which the elastic members are present in the course of putting the diaper on the wearer's body or during actual use of the diaper. It is also contemplated to provide these elastic members associated with the waist regions so as to be spaced from the absorbent structure by given distance. In this case also, such regions in which none of the elastic members is present should be spaced from the wearer's skin. In consequence, according to such a countermeasure, a feeling to wear the diaper should be deteriorated and the diaper should be displaced from the proper position.

The present invention on its first aspect provides a disposable diaper improved so that the liquid-absorbent structure can be put in close contact with the wearer's body in the front waist region without sacrificing the bodily fluid absorbing capacity of the liquid-absorbent structure while it is possible in the rear waist region to assure a space adapted to receive and retain discharged feces without anxiety of unintentionally spreading discharged feces and, in addition, an appropriate fit of the chassis as a whole to the wearer's body can be assured.

### <Second Aspect of the Invention>

The elastic members associated with the waist regions in the diaper disclosed by JP 2002-248127A comprise a plurality of elastic members circumferentially extending completely along respective waist-opening peripheries of the front and rear waist regions and a plurality of elastic members extending from the side edges of the respective waist regions to the side edges of the absorbent structure. In this way, a tensile stress of the waist elastic members is exerted on the side edges of the absorbent structure so as to improve a fit of the absorbent structure to the wearer's body.

However, the side edges of the absorbent structure are put in close contact with the wearer's body under the tensile stress of the elastic members in the rear waist region in the same manner as in the front waist region. As a result, it is difficult to leave a clearance gap between the wearer's body and the absorbent structure and it is still more difficult to assure a space sufficiently large to receive and retain discharged feces without spreading the latter. Eventually, such discharged feces may come in contact with the wearer's buttock, soil the wearer's skin and/or cause eruption.

The present invention on its second aspect provides a disposable diaper improved so that, in the front waist region, the fit of the liquid-absorbent structure to the wearer's body is ensured so as to prevent bodily fluids from leaking sideways but not to deteriorate the bodily fluid absorbing capacity of the liquid-absorbent core and, in the rear waist region, it is assured to leave between the wearer's buttock and the bodily fluid absorbent structure a space adapted to receive and retain discharged feces without spreading the latter.

### MEASURE TO SOLVE THE PROBLEM

According to the present invention on its first aspect, there is provided a disposable diaper comprising:
a chassis having a longitudinal direction, a transverse direction, a side facing the wearer's skin and a side facing away from the wearer's skin and configurationally comprising a front waist region, a rear waist region, a crotch region extending between said front and rear waist regions, a waist-opening and a pair of leg-openings, a liquid-absorbent structure provided on the side facing the wearer's skin of the chassis and extending across the crotch region further into said front and rear waist regions, the liquid-absorbent structure containing therein a liquid-absorbent core, and, in each of the front and rear waist regions, the disposable diaper comprises an upper elasticized region extending in a vicinity of a periphery of the waist-opening, lower elasticized regions, an intermediate elasticized region extending between the upper elasticized region and the lower elasticized regions and outside longitudinally opposite ends of the liquid-absorbent structure, and a non-elasticized region.

According to the present invention on its first aspect is **characterized in that** at least in the front waist region of the front and rear waist regions, the non-elasticized region opposed to a central zone of the liquid-absorbent core wherein a tensile stress of the intermediate elasticized region is lower than a tensile stress of the upper elasticized region.

The present invention on its first aspect may be implemented also in preferred embodiments as follow:
(1) The intermediate elasticized region has a tensile stress lower than a tensile stress of the lower elasticized regions.
(2) The relationship of the tensile stress among the upper elasticized region, the intermediate elasticized region and the lower elasticized region can be represented by the upper elasticized region > the lower elasticized region > the intermediate elasticized region.
(3) The upper elasticized region exhibits a tensile stress in a range of about 1.0 to 3.0 N/25 mm at the state corresponding to about 80% of its maximum extended state, the lower elasticized region exhibits a tensile stress in a range of about 0.5 to 2.0 N/25 mm at the state corresponding to about 80% of its maximum stretched state and the intermediate elasticized region exhibits a tensile stress in a range of about 0.3 to 1.0 N/25 mm at the state corresponding to about 80% of its maximum stretched state.
(4) Tape fasteners for disposal of used diaper are attached to the intermediate elasticized region.

According to the present invention on its second aspect, there is provided a disposable diaper comprising a chassis having a longitudinal direction, a transverse direction, a side facing the wearer's skin and a side facing away from the wearer's skin and configurationally comprising a front waist region, a rear waist region, a crotch region extending between the front and rear waist regions, a waist-opening and a pair of leg-openings, a liquid-absorbent structure provided on the side facing the wearer's skin of the chassis and extending across the crotch region further into the front and rear waist regions, the liquid-absorbent structure containing therein a liquid-absorbent core, and, a plurality of waist elastic members extending under tension in the transverse direction.

The present invention on its second aspect is **characterized in that** the waist elastic members comprise first waist elastic members extending in a transverse direction in a vicinity of the waist-opening periphery and second waist elastic members provided between the leg-openings peripheries and the first waist elastic members and extending in the transverse direction across opposite lateral regions so as not to be present in central zones of the front and rear waist regions, and the second waist elastic members in the front waist region extend from transversely opposite side edges of the front waist region in the transverse direction beyond transversely opposite side edges of the liquid-absorbent core while the second waist elastic members in the rear waist region extend from transversely opposite side edges in the transverse direction and terminate short of the side edges of the liquid-absorbent core.

The present invention on its second aspect may be implemented also in preferred embodiments as follow:
(1) The dimension in the transverse direction between the inner end portions of the second waist elastic members and the side edges of the liquid-absorbent core in the front waist region is in a range of about 10 to 30% of the dimension in the transverse direction of the liquid-absorbent core and the dimension in the transverse direction between the inner end portions of the second waist elastic members and the side edges of the liquid-absorbent core in the front waist region is about 10% or less.
(2) The rear end of the liquid-absorbent core is spaced from the region of the first waist elastic members closest to said rear end.
(3) The chassis includes the sheet lying on the side of the second waist elastic members facing away from the wearer's skin and the inner end portions of the second waist elastic members are permanently bonded to said sheet.
(4) Said sheet is formed of a moisture-permeable plastic film and has a thickness in a range of about 0.015 to 0.05 mm.

### EFFECT OF THE INVENTION

According to the present invention on its first aspect, at least in the front waist region of the front and rear waist regions, none of the elastic members is present in the range opposed to the central zone of the liquid-absorbent core and consequently, the bodily fluid absorbing capacity of the liquid-absorbent core should not be deteriorated under contraction of the elastic members. The tensile stress of the intermediate elasticized region overlying the front and rear ends of the liquid-absorbent core is lower than those of the upper and lower elasticized regions and therefore the chassis as a whole maintains an appropriate fit without sacrificing the bodily fluid absorbing capacity of the liquid-absorbent core.

According to the present invention on its second aspect, in the front waist region, the second waist elastic members extend from the side edges of the front waist region across the side edges of the liquid-absorbent structure to the side edges of the liquid-absorbent core. In the rear waist region, the second waist elastic members extend from the side edges of the rear waist region and terminate short of the side edges of the liquid-absorbent structure, i.e., do not extend to the liquid-absorbent core. With such arrangement, in the front waist region, the tensile stress of the second waist elastic members affects the liquid-absorbent core at a degree not deteriorating the bodily fluid absorbing capacity of the liquid-absorbent core and puts the liquid-absorbent core in close contact with the wearer's body. In the rear waist region, the tensile stress of the second waist elastic members should not directly affect the liquid-absorbent structure so as to put this liquid-absorbent structure in excessively close contact with the wearer's skin. Consequentially, a space sufficiently large to retain the discharged feces without spreading this is assured between the wearer's body and the bodily fluid absorbent structure. In this way, a high retention capability for body waste is obtained.

### BRIEF DESCRIPTION OF THE DRAWINGS

[FIG. 1] Fig. 1 is a partially cutaway perspective view of a diaper according to a first aspect of the invention as partially broken away.
[FIG. 2] Fig. 2 is a developed plan view showing the diaper.
[FIG. 3] Fig. 3 is an exploded perspective view of the diaper.
[FIG. 4] Fig. 4 shows a front member of the diaper in a partially scale-enlarged view A and a rear member of the diaper in a partially scale-enlarged view B.
[FIG. 5] Fig. 5 is a partially cutaway perspective view of a first embodiment of the diaper according to a second aspect of the invention.
[FIG. 6] Fig. 6 is a developed plan view of the diaper.
[FIG. 7] Fig. 7 is an exploded perspective view of the diaper.
[FIG. 8] Fig. 8 shows a front member of the diaper in a partially scale-enlarged view A and a rear member of the diaper in a partially scale-enlarged view B.
[FIG. 9] Fig. 9 is a partially cutaway perspective view of the second embodiment of the diaper according to the second aspect of the invention.

### IDENTIFICATION OF REFERENCE NUMERALS USED IN THE DRAWINGS

- 10: disposable diaper
- 11: chassis
- 12: liquid-absorbent structure
- 13: front waist region
- 14: rear waist region
- 15: crotch region
- 16: waist-opening
- 17: leg-openings
- 18: upper elasticized region
- 19: intermediate elasticized region
- 20A, 20B: lower elasticized regions
- 24: side edges of front waist region
- 25: side edges of rear waist region
- 30: non-elasticized region
- 44: liquid-absorbent core
- 44d: central zone of liquid-absorbent core
- 60: tape fastener for disposal of diaper
- 112: side edges of liquid-absorbent structure
- 210: disposable diaper
- 211: chassis
- 212: liquid-absorbent structure
- 212c: side edges of liquid-absorbent structure
- 213: front waist region
- 214: rear waist region
- 215: crotch region
- 216: waist-opening
- 216a: waist-opening periphery
- 217: leg-openings
- 218: upper elasticized region
- 219: lower elasticized region
- 220: side edges of front and rear waist regions
- 224: side edges of front waist region
- 225: side edges of rear waist region
- 230: non-elasticized region
- 231: first waist elastic member
- 231a: region of first waist elastic member placed aside closely toward rear end of liquid-absorbent core
- 232: second waist elastic member
- 233: leg elastic members
- 238: inner end of second waist elastic member
- 244: liquid-absorbent core
- 244c: side edges of liquid-absorbent core
- 270: (fixing) sheet
- D1: dimension as measured in transverse direction between inner end of second waist elastic member and waist associated side edge of liquid-absorbent core in rear region
- L1: dimension as measured in transverse direction between innerend of second waist elastic member and region associated side edge of liquid-absorbent core in front waist
- L2: dimension of liquid-absorbent core as measured in transverse direction
- W: dimension of liquid-absorbent structure as measured in transverse direction
- X: longitudinal direction
- Y: transverse direction

### DESCRIPTION OF THE BEST MODE FOR WORKING OF THE INVENTION

### <First Aspect of the Invention>

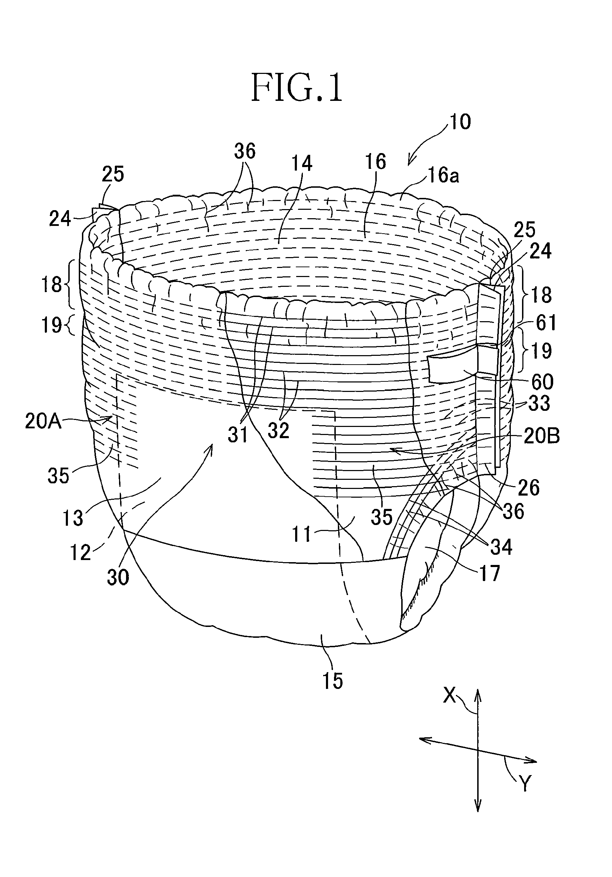

Figs. 1 through 4 illustrate a first embodiment of the present invention on its first aspect. Fig. 1 is a partially cutaway perspective view of a diaper 10 as put on the wearer's body. Referring to Fig. 1 showing the diaper 10 put on the wearer's body wherein a longitudinal direction is designated by X and a transverse direction is designated by Y.

As will be seen in Fig. 1, the diaper 10 comprises a chassis 11 and a liquid-absorbent structure 12 attached to the inner side of the chassis 11, i.e., the side of the chassis 11 facing the wearer's skin and extending in the longitudinal direction X. The diaper 10 configurationally comprises a front waist region 13, a rear waist region 14 and a crotch region 15 extending between these two waist regions. Each of the front and rear waist regions 13, 14 is sectionalized into an upper elasticized region 18, an intermediate elasticized region 19, lower elasticized regions 20A, 20B and a non-elasticized region 30 extending between the lower elasticized regions 20A, 20B. The upper elasticized region 18 is provided with a plurality of first waist elastic members 31 extending along a waist periphery 16a in the transverse direction Y, the lower elasticized regions 20A, 20B are respectively provided with a plurality of third waist elastic members 33 extending in the transverse direction Y across opposite lateral regions of each waist region 13 or 14, and the intermediate elasticized region 19 is provided with a plurality of waist elastic members 32 extending in the transverse direction Y between the first waist elastic members 31 and the third waist elastic members 33. As one variant, it is possible to form the non-elasticized region 30 in the front waist region 13 alone.

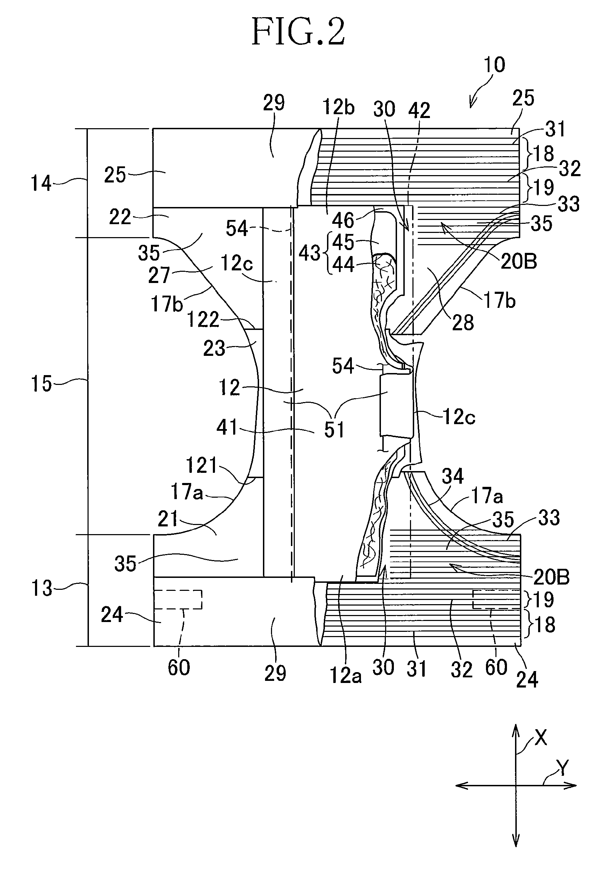

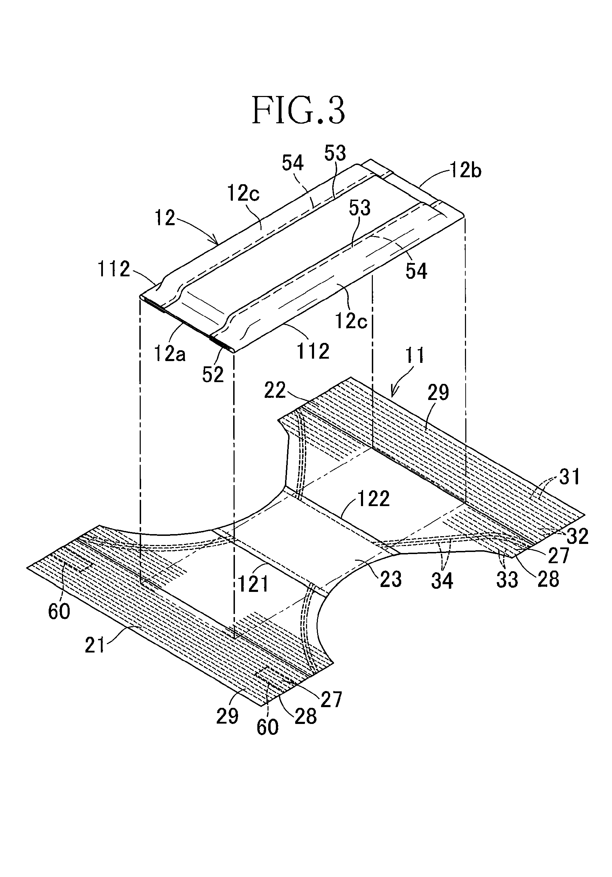

Fig. 2 is a developed plan view of the diaper 10 as the front and rear waist regions 13, 14 have been separated from each other along respective seams 26 and developed in the longitudinal direction X as well as in the transverse direction Y and Fig. 3 is an exploded perspective view of the diaper 10 as the chassis 11 and the liquid-absorbent structure 12 have been separated from each other.

The chassis 11 comprises a substantially trapezoidal front member 21 including the front waist region 13, a substantially trapezoidal rear member 22 including the rear waist region 14 and a substantially rectangular intermediate member 23 including the crotch region 15. These front member 21, intermediate member 23 and the rear member 22 are arranged in the longitudinal direction X in this order and joined together along joint lines 121, 122. Referring again to Fig. 1, respective pairs of transversely opposite side edges 24, 25 of the front member 21 and the rear member 22 are put flat and bonded together at seams 26 arranged intermittently in the longitudinal direction X by means of, for example, hot melt adhesive, heat embossing, sonic or heat sealing technique, whereupon a waist-opening 16 and a pair of leg-openings 17 are defined. The transversely opposite side edges 24 are provided on the outer surface with tape fasteners 60 attached thereto. These tape fasteners are used to fasten the soiled diaper 10 in a rolled up state for disposal.

The front member 21, the intermediate member 23 and the rear member 22 are formed by a liquid-pervious inner layer sheet 27 facing the wearer's skin and a liquid-impervious outer layer sheet 28 facing away from the wearer's skin joined to each other. Both the inner layer sheet 27 and the outer layer sheet 28 are formed from an air-permeable fibrous nonwoven fabric. The outer layer sheet 28 extends in the longitudinal direction X beyond longitudinally opposite ends of the inner layer sheet 27. After the liquid-absorbent structure 12 has been placed on the inner side of the chassis 11, the portions 29 of the outer layer sheet 28 extending outward beyond the opposite ends of the inner layer sheet 27 in this manner are folded back toward the liquid-absorbent structure 12 so as to cover front and rear ends 12a, 12b of the liquid-absorbent structure 12 and bonded thereto at the respective pairs of side edges 24, 25 of the front and rear waist regions 13, 14. The portions 29 of the outer layer sheet 28 covering the front and rear ends 12a, 12b of the liquid-absorbent structure 12 from above function as leak-barrier walls preventing body waste from leaking out even if any amount of bodily fluids which has not been absorbed by the liquid-absorbent structure 12 leaks beyond the front and rear ends 12a, 12b.

Between the inner layer sheet 27 and the outer layer sheet 28, the first waist elastic members 31, the second waist elastic members 32, the third waist elastic members 33 and a plurality of leg elastic members 34 extending along upper and lower halves 17a, 17b of the respective leg-openings' peripheries are interposed. These elastic members 31, 32, 33, 34 are attached under tension at least to the inner layer sheet 27 of the inner layer sheet 27 and outer layer sheet 28 by means of hot melt adhesive (not shown). A plurality of gathers 36 appear in the chassis 11 under contraction of these elastic members 31, 32, 33, 34 (See Fig. 1).

The liquid-absorbent structure 12 has a substantially rectangular shape and comprises a liquid-pervious inner sheet 41, a liquid-impervious outer sheet 42 and a liquid-absorbent core assembly 43 interposed between these two sheets. The inner sheet 41 and the outer sheet 42 extend outward beyond a peripheral edge of the substantially rectangular core assembly 43 and the portions of these sheets 41, 42 extending outward beyond the periphery of the core assembly 43 are put flat and bonded together by means of hot melt adhesive (not shown) to define the front end 12a opposed to the front end of the chassis 11, the rear end 12b opposed to the rear end of the chassis 11 and a pair of opposite side edges 12c extending between the front and rear ends 12a, 12b in the longitudinal direction X. The front end 12a and the rear end 12b are bonded to the inner layer sheet 27 in the front member 21 and to the inner layer sheet 27 in the rear member 22, respectively, by means of hot melt adhesive (not shown).

The core assembly 43 comprises a rectangular liquid-absorbent core 44 formed from a mixture of fluff pulp, super-absorbent polymer particles and, if desired, heat-sealable staple fibers and a liquid-spreadable shape retaining sheet 45 such as tissue paper with which the core 44 is wrapped. Between the outer sheet 42 and the core assembly 43, a liquid-barrier sheet 46 formed from a liquid-impervious and moisture-permeable plastic film is interposed. A size of the liquid-barrier sheet 46 is sufficient to cover most part of the bottom face of the core assembly 43 and preferably covers the entire bottom face of the core assembly 43 in order to ensure a sufficient leak-barrier effect.

A pair of barrier cuffs 51 each formed from a liquid-impervious sheet and extending in the longitudinal direction X are attached to the opposite side edges 12c of the liquid-absorbent structure 12. Each of these barrier cuffs 51 has a fixed edge 52 fixed between the associated side edge 12c of the liquid-absorbent structure 12 and the chassis 11 so as to intersect with the innermost segment of the associated leg elastic members 34 and a free edge 53 collapsed toward the side of the liquid-absorbent core 44 wherein at least a single elastic member 54 extending in the longitudinal direction X is attached to the inner surface of the free edge 53. With the diaper 10 put on the wearer's body, the free edge 53 is spaced upward from the inner sheet 41 under contraction of the elastic member 54 and thereby completely covers the associated side edge 12c of the liquid-absorbent structure 12 to prevent any amount of body waste from leaking sideways. In the crotch region 15, the elastic member 54 extends in the longitudinal direction X and cooperates with the leg elastic members 34 extending along the upper and lower halves 17a, 17b of the associated leg-opening 17 to define an imaginary annular elasticized region along this leg-opening 17. In this way, the imaginary annular elasticized regions surround the wearer' s legs (not shown) and thereby prevent leak of body waste from occurring around the wearer's legs.

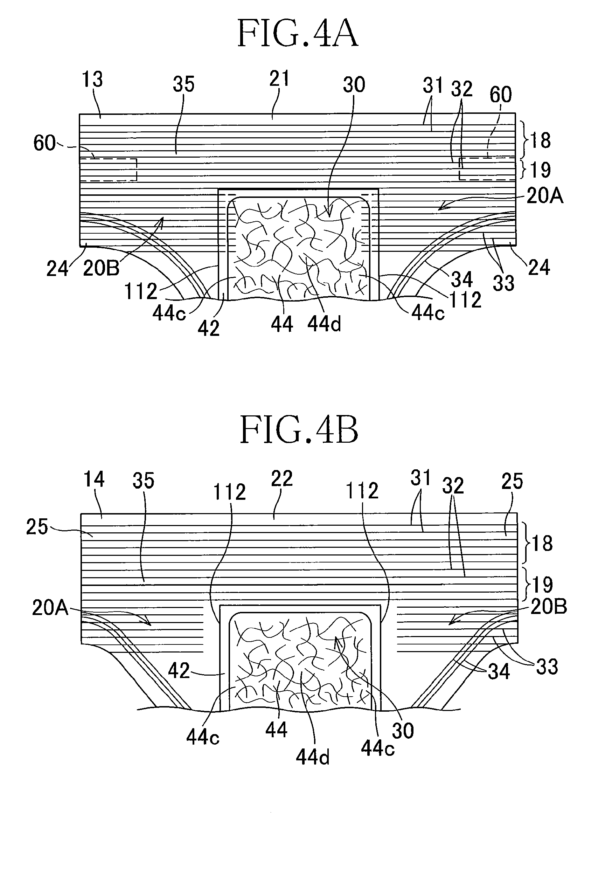

Fig. 4A and 4B are partially scale-enlarged diagrams illustrating the front member 21 and the rear member 22, respectively. For convenience of illustration, the inner layer sheet 27, the liquid-barrier sheet 46, the shape retaining sheet 45 and the inner sheet 41 are eliminated in Fig. 4A and 4B.

In the front waist region 13, as will be apparent from Fig. 4A and 4B, the third waist elastic members 33 extend from the opposite side edges 24 in the transverse direction slightly beyond the opposite side edges 112 of the liquid-absorbent structure 12 to opposite side edges 44c of the liquid-absorbent core assembly 44 and not into the central zone 44d. In the rear waist region 14, the third waist elastic members 33 extend from the opposite side edges 25 and terminate short of the opposite side edges 112 of the liquid-absorbent structure 12.

To assure a desired fit of the liquid-absorbent structure 12 to the wearer's body in the front waist region 13, it is essential that the lower elasticized regions 20A, 20B including the third waist elastic members 33 extending therein should have a correspondingly high tensile stress. However, the intermediate elasticized regions 19 overlying these lower elasticized regions 20A, 20B and including the second waist elastic members 32 extending therein preferably has a tensile stress lower than the tensile stress of the lower elasticized regions 20A, 20B. Specifically, to minimize any affection due to contraction of the waist elastic members 31, 32, 33 exerted on the core 44 and thereby to maintain the desired body fluid absorbing capacity of the core 44, it may be contemplated that the second waist elastic members 32 extending outside the front and rear ends 12a, 12b of the liquid-absorbent structure 12 as viewed in the longitudinal direction X, i.e., extending in the intermediate elasticized region 19 are spaced from the front and rear ends of the liquid-absorbent core 44 by an appropriate distance or the tensile stress of the second elastic members 32 themselves is appropriately reduced. However, if the second waist elastic members are eliminated or spaced in the longitudinal direction X from the liquid-absorbent structure 12 by a given distance, both the front waist region 13 and the rear waist region 14 will be formed with relatively large non-elasticized regions, undesirably resulting in a deteriorated fit of the chassis 11. It may be also contemplated that the intermediate region 19 is provided with the second waist elastic members 32 and these elastic members 32 are partially cut off or removed at locations above the front end 12a of the liquid-absorbent structure 12. However, such measure is inevitably accompanied with an inconvenience, for example, when the waist-opening periphery 16a is pulled up in the course of putting the diaper 10 on the wearer's body, the non-elasticized region including none of the second waist elastic members 32 extending therein stays at its initial level without being pulled up together with the upper elasticized region 18 and the lower elasticized regions 20A, 20B to a desired level. In consequence, this non-elasticized region dropped down between the upper elasticized region 18 and the liquid-absorbent structure 12, affecting not only the feeling to wear but also the appearance. In addition, if bodily fluids are discharged directly onto such dropped down region, such bodily fluids should not be completely absorbed by the liquid-absorbent core and any amount thereof should eventually leak out.

According to the present invention on its first aspect, the intermediate elasticized region 19 including the second waist elastic members 32 extending therein is formed above the front and rear ends 12a, 12b of the liquid-absorbent structure 12 in a manner that the tensile stress of this intermediate elasticized region 19 is adjusted not to affect the liquid absorbing capacity of the liquid-absorbent core 44. In this way, both requirements for the bodily fluid absorbing capacity and the appropriate fitness of the diaper 10 can be satisfied.

To assure a desired fit of the liquid-absorbent structure 12 to the wearer's body, it is required for the lower elasticized regions 20A, 20B to have the correspondingly desired tensile stress. To stabilize the front and rear waist regions 13, 14 of the diaper 10 at desired waist regions of the wearer's body, it is required for the upper elasticized region 18 extending in the transverse direction Y along the waist-opening periphery 16a to have a tensile stress higher than those of the intermediate elasticized region 19 and the lower elasticized regions 20A, 20B. In other words, the respective tensile stress values of the upper elasticized region 18 and the lower elasticized regions 20A, 20B are preferably higher than at least the tensile stress of the intermediate elasticized region 19. More specifically, the upper elasticized region 18, the intermediate elasticized region 19 and the lower elasticized regions 20A, 20B preferably have respective tensile stress values as measured in the transverse direction X in a mutual relationship expressed by the upper elasticized region 18 > the lower elasticized regions 20A, 20B > the intermediate elasticized region 19.

Specifically, in the preferable mutual relationship among the respective elasticized regions, the upper elasticized region 18 should exhibit a tensile stress in a range of about 1.0 to 3.0 N/25 mm at the state corresponding to about 80% of its maximum stretched state, the lower elasticized regions 20A, 20B should exhibit a tensile stress in a range of 0.5 to 2.0 N/25 mm at the state corresponding to about 80% of its maximum stretched state and the intermediate elasticized region 19 should exhibit a tensile stress in a range of about 0.3 to 1.0 N/25 mm at the state corresponding to about 80% of its maximum stretched state. Particularly, if the tensile stress of the intermediate elasticized region 19 is less than about 0.3 N/25 mm, the intermediate elasticized region 19 should drop down between the upper elasticized region 18 and the liquid-absorbent structure 12 as has previously been described.

As has previously been described, the waist elastic members 31, 32, 33 are permanently bonded, under respective tensions given by predetermined extending ratios in the transverse direction Y, to at least to the inner surface of the inner layer sheet 27. Preferably, in the course of bonding these elastic members to the inner layer sheet 27, the first waist elastic members 31 are under a tension given by the extending ratio in a range of about 200 to 350%, the second waist elastic members 32 are under a tension given by the extending ratio in a range of about 160 to 250% and the third waist elastic members 33 are under a tension given by the extending ratio in a range of about 250 to 400%. If the first waist-surrounding elastic members 31 are attached, under a tension given by the extending ratio less than about 200%, to the inner layer sheet 27, it will be difficult to broaden the waist-opening 16 sufficiently to receive the wearer's legs smoothly even if it is tried to pull the waist-opening periphery 16a in the transverse direction Y when the diaper 10 is put on the wearer's body. Practically in consequence, it is difficult to put the diaper 10 on the wearer' s body. The second waist elastic members 32 are preferably attached to the inner layer sheet 27 under a tension given by the extending ratio as low as possible in order to prevent the liquid-absorbent core 44 from being unacceptably contracted. However, if the extending ratio is less than 160%, the desired fit of the intermediate elasticized region 19 to the wearer's body can no more assured and eventually the intermediate elasticized region 19 as a whole should slip down between the upper elasticized region 18 and the liquid-absorbent structure 12.

As will be appreciated from the foregoing description, the extending ratio of the third elastic members 33 may be set to be higher than those of the first and second waist elastic members 31, 32 to ensure that the lower elasticized regions 20A, 20B can be extended depending on the wearer's body shape and in response to the wearer's movement and thereby any significant displacement of the liquid-absorbent structure 12 can be avoided.

The tape fasteners 60 for disposal of the used diaper are preferably attached to the front and rear waist regions 13, 14 in the respective intermediate elasticized regions 19. As has previously been described, the intermediate elasticized region 19 has the tensile stress and the extending ratio both lower than those of the other elasticized regions 18, 20A, 20B and, therefore, fixed regions 61 of the respective tape fasteners are substantially free from contraction of the other regions. In consequence, operation of fastening the used diaper 10 can be smoothly carried out for disposal thereof.

The first waist elastic members 31, the second and third waist members 32, 33 may be made of natural or synthetic rubber and may be implemented in the form of strings, cords or tape having rubber elasticity such as, Lycra (Registered Trademark) as elastic elements. In the case of the illustrated example, in the front waist region 13, the first waist elastic member 31 comprises six elastic elements, the second waist elastic member 32 comprises three elastic elements and the third waist elastic member 33 comprises twelve elastic elements. In the rear waist region 14, the first waist elastic member 31 comprises six elastic elements, the second waist elastic member 32 comprises five elastic elements and the third elastic member 33 comprises seven elastic elements. The number of these elastic elements as well as a distance between each pair of the adjacent elastic elements in the respective elastic members may be appropriately varied depending on the tensile stress and the extending ratio required for the respective elasticized regions 18, 19, 20A, 20B. The lower elasticized regions 20A, 20B in the front waist region 13 preferably have a tensile stress higher than that of the lower elasticized regions 20A, 20B in the rear waist region 14. This is for the reason that, in the front waist region 13, the liquid-absorbent structure 12 must be held in close contact with the wearer's body to prevent urine leak while, in the rear waist region 14, it is essential to assure the space serving to retain discharged feces and the fit may be of a degree sufficient to prevent urine leakage.

While the first waist elastic members 31, the second and third waist elastic members 32, 33 are attached to the respective regions 18, 19, 20A, 20B to elasticize these regions in the illustrated embodiment, it is possible to form the respective elasticized regions 18, 19, 20A, 20B by sheet members themselves having elasticity or by permanently attaching such elastic sheets to the inner surface of the chassis 11.

The tensile stress values of the respective elasticized regions 18, 19, 20A, 20B are measured by a method as will be described below.

First, the diaper 10 is developed as seen in Fig. 2 and the respective waist elastic members 31, 32, 33 are extended. Test pieces each having a width of about 25 mm for the respective elasticized regions 18, 19, 20A, 20B are cut away from the diaper 10 in this extended state and this length of about 25 mm is the maximum extended length. The test piece is held by chucks of a contractile strength tester and subj ected to 1 cycle test with a moving velocity of 100 mm/min and an inversion distance corresponding to 90% of the maximum extended length of the test piece. A tensile stress after inversion is measured at a point the length of the test piece reaching 80% of its maximum extended length. It should be noted that a distance between the chucks should be appropriately changed depending on the test piece and, when it is difficult to obtain the test piece having a width of 25 mm, the measurement may be carried out on the basis of the test piece having an optional width and the measurement result may be converted into a tensile stress value to be obtained on the basis of the test piece having a width of 25 mm.

The components constituting the diaper 10 of the invention such as the inner and outer layer sheets 27, 28, the inner and outer sheets 41, 42 and the liquid-absorbent structure 12 may be formed by those conventionally used in the relevant field used to make the disposable diaper. While the pants-type disposable diaper 10 comprising the front and rear waist regions joined together along the respective pairs of opposite side edges 24, 25 are illustrated and described as the embodiment, the present invention is applicable not only to the pants-type diaper but also to the open-type diaper. It is also possible to form the front member 21 and the intermediate member 23 of the chassis 11 by a continuous sheet or to form the chassis 11 by the front member 21 and the rear member 22 without incorporation of the intermediate member 23.

### <Second Aspect of the Invention>

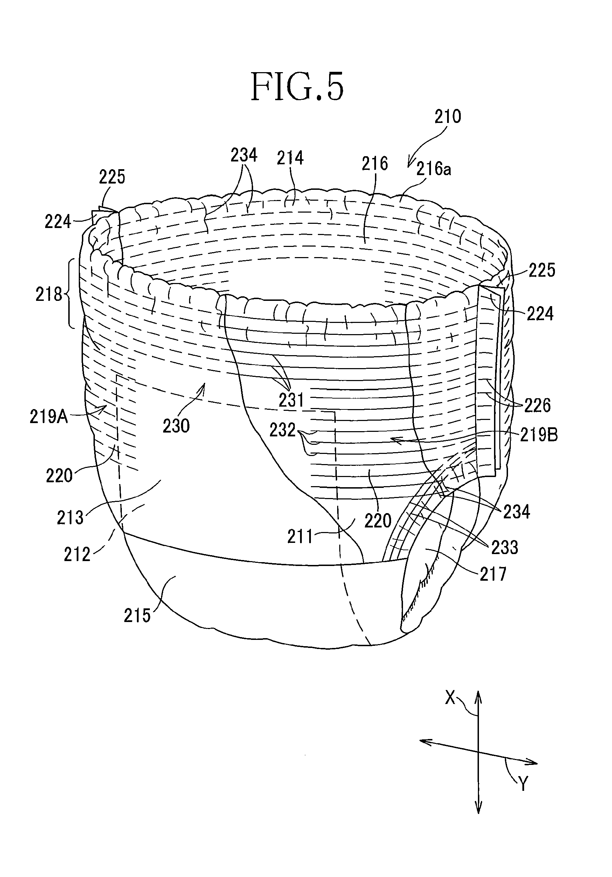

Figs. 5 through 9 illustrate a first embodiment of the present invention on its second aspect. Fig. 5 is a partially cutaway perspective view of a diaper 210 as put on the wearer's body. Referring to Fig. 5 showing the diaper 210 put on the wearer's body wherein a longitudinal direction is designated by X and a transverse direction is designated by Y.

As will be seen in Fig. 5, the diaper 210 comprises a chassis 211 and a liquid-absorbent structure 212 attached to the inner side of the chassis 211, i.e. , the side of the chassis 211 facing the wearer's skin and extending in the longitudinal direction X. The diaper 10 configurationally comprises a front waist region 213, a rear waist region 214 and a crotch region 215 extending between these two waist regions. Each of the front and rear waist regions 213, 214 is sectionalized into an upper elasticized region 218, lower elasticized regions 219A, 219B and a non-elasticized region 230 extending between the lower elasticized regions 219A, 219B. The upper elasticized region 218 is provided with a plurality of first waist elastic member 231 extending along a waist-opening periphery 216a in the transverse direction Y and the lower elasticized regions 219A, 219B are respectively provided with a plurality of second waist elastic member 232 extending in the transverse direction Y across opposite lateral regions 220 of each waist region 213, 214.

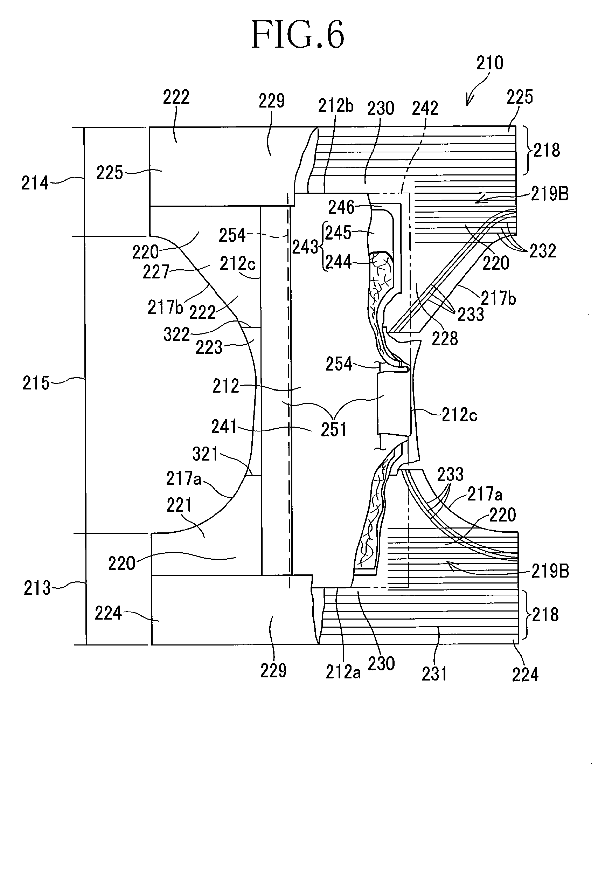

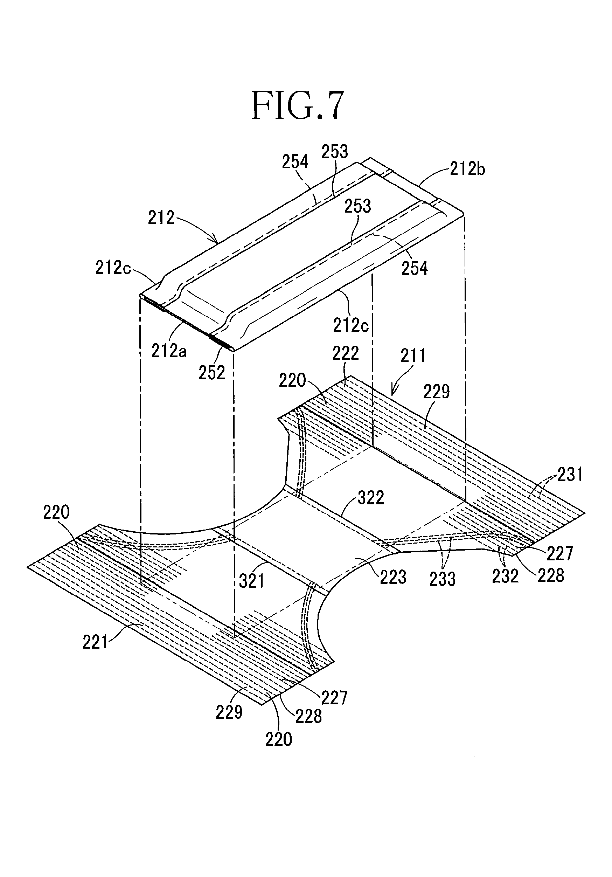

Fig. 6 is a developed plan view of the diaper 210 as the front and rear waist regions 213, 214 have been separated from each other along respective seams 226 and developed in the longitudinal direction X as well as in the transverse direction Y and Fig. 7 is an exploded perspective view of the diaper 210 as the chassis 211 and the liquid-absorbent structure 212 have been separated from each other.

The chassis 211 comprises a substantially trapezoidal front member 221 including the front waist region 213, a substantially trapezoidal rear member 222 including the rear waist region 214 and a substantially rectangular intermediate member 223 extending between the front member 221 and the rear member 222 and including the crotch region 215. These front member 221, intermediate member 223 and the rear member 222 are arranged in the longitudinal direction X in this order and joined together along joint lines 321, 322. Respective pairs of transversely opposite side edges 224, 225 of the front member 221 and the rear member 222 are put flat and bonded together at seams 226 arranged intermittently in the longitudinal direction X by means of, for example, hot melt adhesive, heat embossing, sonic or heat sealing technique, whereupon a waist-opening 216 and a pair of leg-openings 217 are defined. The transversely opposite side edges 24 are provided on the (See Fig. 1).

The front member 221, the intermediate member 223 and the rear member 222 are formed by a liquid-pervious inner layer sheet 227 facing the wearer's skin and a liquid-impervious outer layer sheet 228 facing away from the wearer's skin joined to each other. Both the inner layer sheet 227 and the outer layer sheet 228 are formed from an air-permeable fibrous nonwoven fabric. The outer layer sheet 228 extends in the longitudinal direction X beyond longitudinally opposite ends of the inner layer sheet 227. After the liquid-absorbent structure 212 has been placed on the inner side of the chassis 211, the portions 229 of the outer layer sheet 228 extending outward beyond the opposite ends of the inner layer sheet 227 in this manner are folded back toward the liquid-absorbent structure 212 so as to cover front and rear ends 212a, 212b of the liquid-absorbent structure 212 and bonded thereto at the respective pairs of side edges 224, 225. The portions 229 of the outer layer sheet 228 covering the front and rear ends 212a, 212b of the liquid-absorbent structure 212 from above function as leak-barrier walls preventing body waste from leaking out even if any amount of bodily fluids which has not been absorbed by the liquid-absorbent structure 212 leaks beyond the front and rear ends 212a, 212b.

Between the inner layer sheet 227 and the outer layer sheet 228, the first waist elastic members 231, the second waist elastic members 232 and a plurality of leg elastic members 233 extending along upper and lower halves 217a, 217b of the respective leg-openings' peripheries are sandwiched. These elastic members 231, 232, 233 are attached under tension at least to the inner layer sheet 227 of the inner layer sheet 227 and outer layer sheet 228 by means of hot melt adhesive (not shown). A plurality of gathers 234 appear in the chassis 211 under contraction of these elastic members 231, 232, 233 (See Fig. 1).

The liquid-absorbent structure 212 has a substantially rectangular shape and comprises a liquid-pervious inner sheet 241, a liquid-impervious outer sheet 242 and a liquid-absorbent core assembly 243 interposed between these two sheets. The inner sheet 241 and the outer sheet 242 extend outward beyond a peripheral edge of the substantially rectangular core assembly 243 and the portions of these sheets 241, 242 extending outward beyond the periphery of the core assembly 243 are put flat and bonded together by means of hot melt adhesive (not shown) to define the front end 212a opposed to the front end of the chassis 211, the rear end 212b opposed to the rear end of the chassis 211 and a pair of opposite side edges 212c extending between the front and rear ends 212a, 212b in the longitudinal direction X. The front end 212a and the rear end 212b are bonded to the inner layer sheet 227 in the front member 221 and to the inner layer sheet 227 in the rear member 222, respectively, by means of hot melt adhesive (not shown).

The core assembly 243 comprises a rectangular liquid-absorbent core 244 formed by a mixture of fluff pulp, super-absorbent polymer particles and, if desired, heat-sealable staple fibers and a liquid-spreadable shape retaining sheet 245 such as tissue paper with which the core 244 is wrapped. Between the outer sheet 242 and the core assembly 243, a liquid-barrier sheet 246 formed from a liquid-impervious and moisture-permeable plastic film is interposed. A size of the liquid-barrier sheet 246 is sufficient to cover most part of the bottom face of the core assembly 243 and preferably covers the entire bottom face of the core assembly 243 in order to ensure a sufficient leak-barrier effect.

A pair of barrier cuffs 251 each formed from a liquid-impervious sheet and extending in the longitudinal direction X are attached to the opposite side edges 212c of the liquid-absorbent structure 212. Each of these barrier cuffs 251 has a fixed edge 252 fixed between the associated side edge 212c of the liquid-absorbent structure 212 and the chassis 211 so as to intersect with the innermost segment of the associated leg elastic members 233 and a free edge 253 collapsed toward the side of the liquid-absorbent core 244 wherein at least one elastic member 254 extending in the longitudinal direction X is attached to the inner surface of the free edge 253. With the diaper 210 put on the wearer's body, the free edge 253 is spaced upward from the inner sheet 241 under contraction of the elastic member 254 and thereby completely covers the associated side edge 212c of the liquid-absorbent structure 212 to prevent any amount of body waste from leaking sideways. In the crotch region 215, the elastic member 254 extends in the longitudinal direction X and cooperates with the leg elastic members 233 extending along the upper and lower halves 217a, 217b of the associated leg-opening 217 to define an imaginary annular elasticized region along this leg-opening 217. In this way, the imaginary annular elasticized regions surround the wearer's legs (not shown) and thereby prevent leak of body waste from occurring around the wearer's legs.

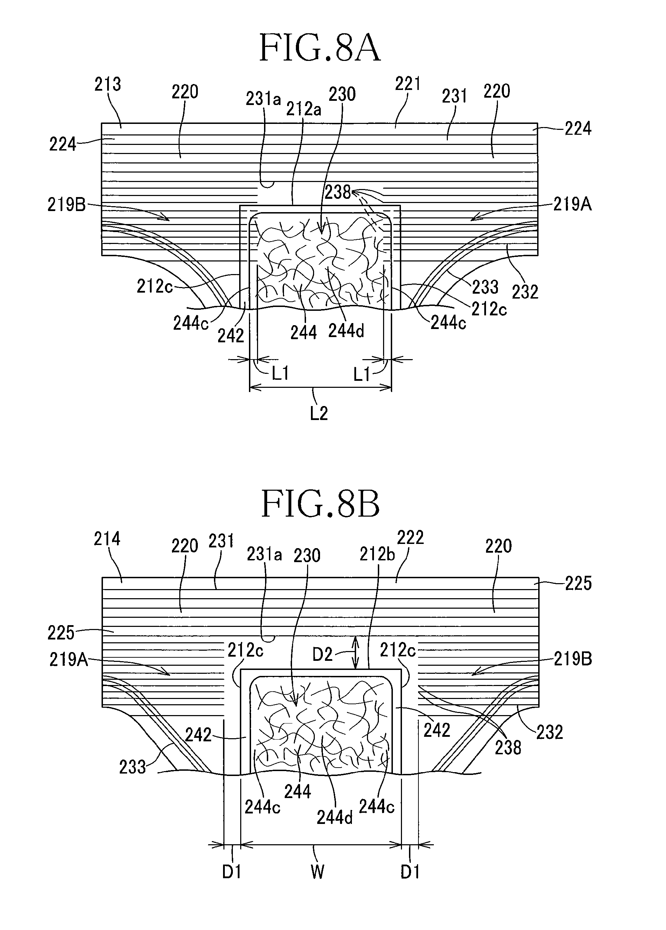

Fig. 8A is a partially scale-enlarged diagram illustrating the front member 221 which has been illustrated also on Fig. 6 in the developed plan view of the diaper 210. For convenience of illustration, the inner layer sheet 227, the liquid-barrier sheet 246, the shape retaining sheet 245 and the inner sheet 241 are eliminated in Fig. 8.

As will be apparent from Fig. 8A, the second waist elastic members 232 extend from the side edges 224 toward the liquid-absorbent structure 212 and extend inward slightly beyond the side edges 212c of the liquid-absorbent structure 212 to the side edges 244c of the core 244. The second waist elastic members 232 extend in this manner to the side edges 244c of the core 244 but not to the central zone 244d of the core 244 so that the core 244 as a whole is substantially free from any affection due to contraction of the second waist elastic members 232 and, in consequence, the liquid-absorbent structure 212 is able to maintain not only its bodily fluid absorbing capacity but also its fit to the wearer's body.

Assumed that the second waist elastic members 232 extend under tension across the liquid-absorbent structure 212 in the transverse direction Y, the liquid-absorbent structure 212 will be affected by contraction of the second waist elastic members 232 and the core 244 as a whole will get wrinkled, resulting in that the bodily fluid absorbing capacity of the core 244 should be significantly deteriorated. On the contrary, when the second waist elastic members 232 extend from the respective pairs of opposite side edges 224, 225 of the front and rear waist regions 213, 214 and terminate short of the side edges 212c of the liquid-absorbent structure 212, i.e., the second waist elastic members are not present on the core 244, the tensile stress of the second waist elastic members 232 should excessively affect the liquid-absorbent structure 212 and therefore the core 244 should not get wrinkles.

However, if the liquid-absorbent structure 212 is bonded to the inner surface of the chassis 211 only at the front and rear ends 212a, 212b and the tensile stress of the second elastic members 232 is not exerted on the liquid-absorbent structure 212, the liquid-absorbent structure 212 should be forced up forward together with the wearer' s front waist region as the wearer bends him- or herself forward and consequently the liquid-absorbent structure 212 should be slipped up from the desired position. In order to avoid such displacement of the liquid-absorbent structure 212, it may be contemplated to coat the large surface of the liquid-absorbent structure 212 facing away from the wearer's skin and facing the chassis 211 with adhesive. In this case, the moisture-permeability of the diaper 210 as a whole is deteriorated and the interior of the diaper becomes stuffy. This is undesirable from the hygiene viewpoint.

To overcome this problem, in the front waist region 213, the second waist elastic members 232 extend to the side edges 244c of the liquid-absorbent core 244 and not to the central zone 244d of the liquid-absorbent core 244 so that the absorbing capacity of the core 244 may be substantially not affected by the second waist elastic members 232. The central zone 244d of the core 244 is not affected by contraction of the second waist elastic members 232 and therefore not apt to get wrinkles. In this way, the absorbing capacity of the core 244 is substantially not deteriorated. On the other hand, the side edges 244c are put in close contact with the wearer' s body under the tensile stress of the second waist elastic members 232 and thereby any displacement of the liquid-absorbent structure 212 is also restrained.

To achieve such effect, a distance L1 in the transverse direction Y between the side edges of the core 244 and the associated inner end portions 238 of the second waist elastic members 238 is preferably about 10 to 30% of a dimension L2 in the transverse direction Y of the liquid-absorbent core 244. If the distance L1 is about 30% or more of the dimension L2 in the transverse direction Y of the liquid-absorbent core 244, the core 244 will get wrinkles over a relatively large area under contraction of the second waist elastic members 232, resulting in deterioration of the bodily fluid absorbing capacity of the core 244 and occurrence of liquid leak. If the distance L1 is less than 10% of the dimension L2 in the transverse direction Y of the liquid-absorbent structure 212, the tensile stress of the second waist elastic members 232 will not effectively function to put the liquid-absorbent structure in close contact with the wearer's body, resulting in that a clearance gap will be formed between the liquid-absorbent structure 212 and the wearer's body and bodily fluids should leak out through such clearance gap.

Fig. 8B is a partially scale-enlarged diagram illustrating the rear member 222 which has been illustrated also by Fig. 6 in the developed plan view of the diaper 210. For convenience of illustration, the inner layer sheet 227, the liquid-barrier sheet 246, the shape retaining sheet 245 and the inner sheet 241 are eliminated in Fig. 8 as is the case with Fig. 8A.

Referring to Fig. 8B, the second waist elastic members 232 extend from the side edges 225 of the rear member 222 in the transverse direction Y and terminate short of the side edges 212c of the liquid-absorbent structure 212. Unlike in the front member 221, the second waist elastic members 232 do not extend to the liquid-absorbent structure 212 and, in consequence, the liquid-absorbent structure 212 is substantially not affected by contraction of the second waist elastic members 232. In this way, between the liquid-absorbent structure 212 and the wearer's buttock, the space to retain discharged feces without spreading this can be assured.

Assumed now that, similar to the case of the front waist region 213, the second waist elastic members 232 extend in the transverse direction Y slightly beyond the side edges 212c of the liquid-absorbent structure 212 in the rear waist region 214 also, the liquid-absorbent structure 212 as a whole will be put in close contact with the wearer' s buttock and it will be difficult to assure the space to retain discharged feces. As a result, the discharged feces will be squeezed between the liquid-absorbent structure 212 and the wearer's buttock and the feces will soil the wearer's skin or leak out from the diaper. However, in the rear waist region 214 according to this embodiment of the invention, the affection due to contraction of the second waist elastic members 232 is minimized so that the liquid-absorbent structure 212 is not put in excessively close contact with the wearer's buttock and the space of an appropriate size to retain the discharged feces is defined between the buttock and the liquid-absorbent structure 212.

In the rear waist region 214, a distance D1 in the transverse direction Y between the inner ends 238 of the second waist elastic members 232 and the side edges 212c of the liquid-absorbent structure 212 is preferably less than 10% of a dimension W in the transverse direction Y of the liquid-absorbent structure 212. This is for the reason that, if the distance D1 is about 10% or more of the dimension W in the transverse direction Y of the liquid-absorbent structure 212, the fit of the liquid-absorbent structure 212 to the wearer's body will be seriously deteriorated and the space defined between the liquid-absorbent structure 212 and the wearer's buttock will become relatively large, causing a problem that body waste should leak out from the diaper.

While the second waist elastic members 232 may be formed by placing the elastic members each previously extended to the predetermined dimension on transversely opposite lateral portions of the front and rear members 221, 222, it is also possible to form these second waist elastic members 232 by bonding the second waist elastic members 232 under tension in the transverse direction Y and then removing portions of the second waist elastic members 232 lying in a desired range in the vicinity of the central zones of the front and rear waist regions 213, 214 (at least a range opposed to the central zone 244d of the core 244) or using said desired range as a adhesion-free zone and partially cutting this adhesion-free zone so as to snap back this adhesion-free zone (i.e., causes such free zone to contract by itself to the vicinity of the bonded portions of the second waist elastic members 232). In general, during a process for making the disposable diaper, the components such as sheet members and elastic members are successively overlapped one on another in a given direction to assemble these components. In view of this, the latter procedure is preferred.

Between the lower elasticized regions 219A, 219B in the front and rear waist regions 213, 214, the non-elasticized regions 230 including none of the second waist elastic members 232 extending therein is not limited to the regions opposed to the core 244 and present outside the front and rear ends 212a, 212b of the liquid-absorbent structure 212 as viewed in the longitudinal direction X. As has previously been described, not only in the rear member 223 but also in the front member 221, it is essential to alleviate the tensile stress of the second waist elastic members 232 exerted on the central zone 244d of the core 244 and thereby to maintain the body fluid absorbing capacity thereof. Specifically, the portions of the second waist elastic members 232 lying outside the front and rear ends 212a, 212b as viewed in the longitudinal direction X may be cut to ensure that the front and rear ends 212a, 212b are sufficiently spaced from the elastic members closest thereto in the longitudinal direction X (the lowermost elastic member 231a of the first waist elastic members in Fig. 8A and 8B). In consequence, the affection due to contraction of the second waist elastic members 232 can be alleviated. A distance D2 between the rear end 212b of the liquid-absorbent structure 212 and the elastic member 231a is preferably equal to or larger than a distance D1 between the inner end portion 238 of the second waist elastic members 232 and the side edges 212c of the liquid-absorbent structure 212 as viewed in the transverse direction Y. In this case, around the rear end 212b of the liquid-absorbent structure 212, a non-elasticized region having a desired size is defined and allows the discharged feces to be reliably retained.

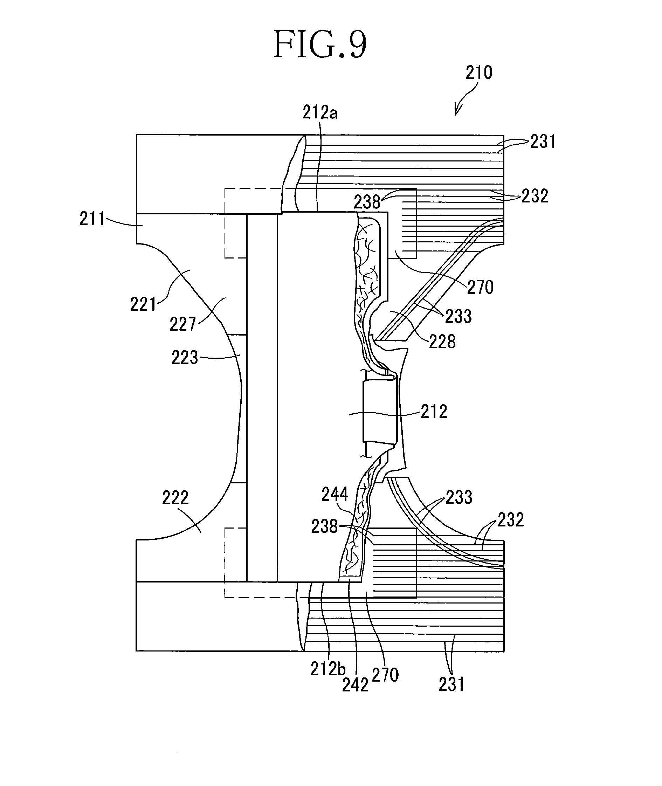

Fig. 9 is a partially cutaway developed plan view showing a second embodiment of the diaper 210 according to the present invention on its second aspect. The diaper 210 according to the second embodiment is basically similar to the first embodiment and therefore only the feature distinguished from the first embodiment will be described here.

Referring to Fig. 9, substantially rectangular moisture-permeable fixing sheets 270 is interposed between the inner layer sheet 227 and the outer layer sheet 228 at a position opposed to the front and rear ends 212a, 212b of the liquid-absorbent structure 212 and bonded to at least respective inner surfaces of the inner layer sheets 227. The inner ends 238 of the second waist elastic members 232 interposed between the inner and outer layer sheets 227, 228 and the vicinity thereof are interposed between the inner layer sheet 227 and the fixing sheets 270 and permanently bonded to the both sheets 227, 270.

While, in many cases, the elastic members are permanently bonded to the sheet members by means of adhesive of well known type such as hot melt adhesive, when the end portions of the elastic members must be bonded under tension to the sheet member as in the case of the second waist elastic members 232, it is required to coat these end portions and the vicinity thereof with much more amount of adhesive than the other joint regions in order to prevent some of the elastic members from falling off during the process of making the diaper or use of the diaper. The regions coated with excessive amount of adhesive inevitably become hardened and deteriorate a unique texture of the fibrous nonwoven fabric, eventually creating a feeling of discomfort against the wearer. In addition, in the case of the diaper in which the outer surface of the outer layer sheet 228 is printed with a figure or a pattern for decorative effect, the regions excessively coated with adhesive may be seen through from the exterior and such decorative effect may be seriously affected.

In the present embodiment, however, the fixing sheets 270 is interposed between the inner and outer layer sheets 227, 228 so as to cooperate with the inner layer sheet 227 to interpose the inner end portions 238 as the joint ends of the second waist elastic members 232. With such arrangement, even if the inner end portions 238 has been coated with a large amount of adhesive, the outer sheet 228 should not be partially hardened and the texture thereof should not be deteriorated. In the course of making the diaper 210, the regions in which the fixing sheets 270 are provided has its thickness as well as stiffness increased in comparison to the other regions. In consequence, the regions of the fixing sheets 270 can be efficiently press-processed and thereby the inner end portions 238 and the vicinity thereof can be further firmly bonded between the inner sheet 227 and the fixing sheets 270. The inner end portions 238 and the vicinity thereof may be further firmly bonded in this way to decrease the amount of adhesive to be used.

The fixing sheet 270 is made of moisture-permeable plastic film or nonwoven fabric and preferably has a thickness in a range of about 0.015 to 0.05 mm. The fixing sheet 270 may have a size at least covering the inner end portion 238 of the second waist elastic member 232 plus about 1 to 20 mm around the end portion 238. The shape of fixing sheet 270 is not limited to the substantially rectangular shape as illustrated, but may have other various shapes as circular or polygonal shape.

It is also possible to interposed two fixing sheets 270 are interposed between the inner and outer layer sheets 227, 228 so as to interpose the inner end portions 238 of the second waist elastic members 232 and the vicinity thereof from above and below. In the case of the fixing sheet 270 comprises upper and lower sheets, the adhesive used to fix the inner end portions 238 and the vicinity thereof may be coated on opposed inner surfaces of these two fixing sheets 270 and consequentially there is no anxiety that the inner layer sheet 227 and the outer layer sheet 228 might be partially hardened due to the presence of the adhesive.

The fixing sheet 270 may be provided with a decorative figure or pattern adapted to be seen through from the exterior by means of printing or the other technique.

The first and second waist elastic members 231, 232, the leg elastic members 233 and elastic members 254 are made of natural or synthetic rubber and implemented in the form of strings or ribbons. The second waist elastic members 232 preferably exhibit a tensile stress in a range of about 0.5 to 2.5 N/25 mm at a extending ratio of about 80%.

The tensile stress values of the second waist elastic members 232 are measured by a method as will be described below.

First, the diaper is developed as seen in Fig. 6 and the second waist elastic members 232 are extended. Test pieces each having a width of about 25 mm for the second waist elastic members 232 are cut away from the diaper 210 in this extended state and this length of about 25 mm is the maximum extended length. The test piece is held by chucks of a contractile strength tester and subjected to 1 cycle test with a moving velocity of 100 mm/min and an inversion distance corresponding to 90% of the maximum extended length of the test piece. A tensile stress after inversion is measured at a point the length of the test piece reaching 80% of its maximum extended length. It should be noted that a distance between the chucks should be appropriately changed depending on the test piece and, when it is difficult to obtain the test piece having a width of 25 mm, the measurement may be carried out on the basis of the test piece having an optional width and the measurement result may be converted into a tensile stress value to be obtained on the basis of the test piece having a width of 25 mm.

While the number of the second waist elastic members 232 as well as the intervals at which these elastic members are arranged in the front and rear waist regions 213, 214 may be appropriately selected depending on the desired tensile stress thereof, the tensile stress of the second waist elastic members 232 as a whole in the front waist region 213, i.e. , the tensile stress of the lower elasticized regions 219A, 219B is preferably higher than the tensile stress of the upper elasticized region 218. It is for the reason that, in the front waist region 213, the liquid-absorbent structure 212 must be held in close contact with the wearer's body to prevent urine leak and, in the rear waist region 214, it is essential above all to assure an appropriate space adapted to retain discharged feces therein with the fitness of a degree sufficient to prevent leak of discharged feces. To ensure that the lower elasticized regions 219A, 219B have the tensile stress higher than the tensile stress of the upper elasticized region 218, the number of the elastic members may be increased in the front waist region 213 compared to the rear waist region 214 so far as the respective elastic members have the same tensile stress. If the number of the elastic members in the front waist region 213 is the same as the number of the elastic members, the elastic members each having the tensile stress higher than the tensile stress of the elastic members in the rear waist region 214 may be provided in the front waist region 213.

The components constituting the diaper 210 of the invention such as the inner and outer layer sheets 227, 228, the inner and outer sheets 241, 242, the respective members constituting the liquid-absorbent structure 212 and the respective elastic members 231, 232, 233, 254 may be formed by those conventionally used in the relevant field used to make the disposable diaper. It is also possible to form the front member 221 and the intermediate member 223 of the chassis 211 by a continuous sheet or to form the chassis 211 by the front member 221 and the rear member 222 without incorporation of the intermediate member 223. While the pants-type disposable diaper 210 comprising the front and rear waist regions joined together along the respective pairs of opposite side edges are illustrated and described as the embodiment, the present invention is applicable not only to the pants-type diaper but also to the open-type diaper.

## Claims

1. A disposable diaper comprising:
a chassis having a longitudinal direction, a transverse direction, a side facing the wearer's skin and a side facing away from the wearer's skin and configurationally comprising a front waist region, a rear waist region, a crotch region extending between said front and rear waist regions, a waist-opening and a pair of leg-openings,
a liquid-absorbent structure provided on said side facing the wearer' s skin of said chassis and extending across said crotch region further into said front and rear waist regions, said liquid-absorbent structure containing therein a liquid-absorbent core, and,
in each of said front and rear waist regions, said disposable diaper comprises an upper elasticized region 18 extending in a vicinity of a periphery of said waist-opening, lower elasticized regions, intermediate elasticized region extending between the upper elasticized region and the lower elasticized regions and outside longitudinally opposite ends of the liquid-absorbent structure, and a non-elasticized region,
said disposable diaper being **characterized in that** at least in the front waist region of the front and rear waist regions, said non-elasticized region opposed to a central zone of said liquid-absorbent core wherein a tensile stress of the intermediate elasticized region is lower than a tensile stress of the upper elasticized region.

2. A disposable diaper comprising
a chassis having a longitudinal direction, a transverse direction, a side facing the wearer's skin and a side facing away from the wearer's skin and configurationally comprising a front waist region, a rear waist region, a crotch region extending between said front and rear waist regions, a waist-opening and a pair of leg-openings,
a liquid-absorbent structure provided on said side facing the wearer's skin of said chassis and extending across said crotch region further into said front and rear waist regions, said liquid-absorbent structure containing therein a liquid-absorbent core, and,
a plurality of waist elastic members extending under tension in said transverse direction,
said disposable diaper being **characterized in that**:
said waist elastic members comprise first waist elastic members extending in a transverse direction in a vicinity of said waist-opening periphery and second waist elastic members provided between said leg-openings peripheries and said first waist elastic members and extending in said transverse direction across opposite lateral regions so as not to be present in central zones of said front and rear waist regions, and
said second waist elastic members in said front waist region extend from transversely opposite side edges of said front waist region in said transverse direction beyond transversely opposite side edges of said liquid-absorbent core while said second waist elastic members in said rear waist region extend from transversely opposite side edges in said transverse direction and terminate short of said side edges of said liquid-absorbent core.
